Europäisches Patentamt

European Patent Office

Office européen des brevets

(11) Veröffentlichungsnummer: **0 135 617**
**B1**

# EUROPÄISCHE PATENTSCHRIFT

(45) Veröffentlichungstag der Patentschrift:
**01.06.88**

(21) Anmeldenummer: **83112623.0**

(22) Anmeldetag: **15.12.83**

(51) Int. Cl.⁴: **A 61 K 31/405**, A 61 K 9/06

(54) **Indomethacin enthaltende, gelartige Salbe.**

(30) Priorität: **29.08.83 CH 4726/83**

(43) Veröffentlichungstag der Anmeldung:
**03.04.85 Patentblatt 85/14**

(45) Bekanntmachung des Hinweises auf die Patenterteilung:
**01.06.88 Patentblatt 88/22**

(84) Benannte Vertragsstaaten:
**AT BE DE FR GB IT LU NL SE**

(56) Entgegenhaltungen:
**EP - A - 0 055 029**
**EP - A - 0 063 870**
**AT - B - 360 654**
**DE - A - 2 827 018**
**DE - A - 2 854 221**
**DE - A - 3 006 024**
**DE - B - 2 103 833**
**GB - A - 2 093 693**
**US - A - 4 029 798**

**M. GLOOR, "Pharmakologie dermatologischer Externa", Springer Verlag, Berlin 1982, Seiten 7, 8 und 42**

(73) Patentinhaber: **Mepha AG, Dornacherstrasse 114, CH-4147 Aesch (CH)**

(72) Erfinder: **Seth, Pyare, Krebsenbachweg 8, CH-4147 Aesch (CH)**

(74) Vertreter: **Frossard, Michel, Dr. et al, A. Braun, Braun, Héritier, Eschmann AG, Patentanwälte Holbeinstrasse 36-38, CH-4051 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Es werden schon seit längerer Zeit Untersuchungen durchgeführt, um zur lokalen Behandlung von Entzündungen ein Präparat auf der Basis von Indomethacin zu schaffen. Der erforderlichen Wirksamkeit eines solchen Präparates stehen nämlich einerseits die schwache Löslichkeit des Wirkstoffes in Wasser und den üblichen Medien, andererseits seine fehlende Absorption durch die menschliche Haut aus den herkömmlichen Salbengrundlagen entgegen.

Um diese Schwierigkeiten zu überwinden, wurde zunächst in der deutschen Patentschrift 2 103 833 die Verwendung von Methylsalicylat als Träger und Lösungsvermittler vorgeschlagen; es soll zugleich die Absorption verstärken. Ein entsprechendes Präparat besteht zum Beispiel aus

50% Äthanol
10% Methylsalicylat
15% Polysorbat 80 ®
 5% Diisopropyladipat
20% Wasser

und enthält 2,0 mg Indomethacin pro Milliliter Methylsalicylat.

In eine andere Richtung weisen die deutschen Offenlegungsschriften 2 827 018 und 3 006 024, gemäss welchen als Träger und Lösungsvermittler jeweils Glykol oder ein Polyalkylenglykol verwendet wird. In der zweitgenannten DE-OS besteht der Träger aus einer überwiegenden Menge eines Polyalkylenglykols und einer geringeren Menge eines Polyäthylenglykol($C_{10}$-$C_{14}$alkyl) äthers. Ein typisches Präparat dieser Art enthält im wesentlichen, nebst dem Indomethacin, Polyäthylenglykole, Polyäthylenglykoldodecyläther und Wasser im Gewichtsverhältnis 75:5:7,4 für eine Salbe bzw. 20:2:73 für ein Gel; zur Bildung des Gels werden Polyacrylsäure und Triäthanolamin verwendet.

Ebenfalls ein Gel wird in der DE-OS 2 827 018 beschrieben; das Trägermedium besteht aus einem Glykol, insbesondere Propylenglykol, Butylenglykol oder Polyäthylenglykol, einem Alkohol und Wasser. Als Geliermittel dienen Cellulose und Cellulosederivate oder das Aminsalz eines Carboxyvinylpolymeren (Polyacrylsäure). Ein solches Gel besteht zum Beispiel aus

| | |
|---|---|
| Indomethacin | 1,0 g |
| Carboxyvinylpolymer | 1,0 g |
| Hydroxyäthylcellulose | 1,0 g |
| Polyäthylenglykol 300 | 10,0 g |
| Äthanol | 30,0 g |
| Diisopropyladipat | 2,0 g |
| Diisopropylamin | 0,9 g |
| Wasser | 54,1 g |

Ein anderes Gel, das zum Einführen in das Rektum, die Vagina und die Urethra bestimmt ist, wird gemäss AT 360 354 aus einem Wirkstoff, beispielsweise Indomethacin, einem Carboxyvinylpolymeren und einer wasserlöslichen basischen Verbindung hergestellt. Vorzugsweise wird ein Löslichmacher wie Propylenglykol oder Polyäthylenglykol verwendet, um die Zusammensetzung durchsichtig zu machen oder die Absorption im Körper zu beschleunigen; am zweckmässigsten ist Propylenglykol wegen seiner universellen Verwendbarkeit.

Von einem in Wasser schwer löslichen Wirkstoff, beispielsweise Indomethacin, wird ebenfalls eine gute Absorption durch die Haut erreicht, wenn er in Form einer Creme gemäss EP 63 870 formuliert wird. Die Creme enthält ein Carboxyvinylpolymeres, Wasser und das Triglycerid einer Fettsäure von mittlerer Kettenlänge ($C_6$-$C_{12}$). Gewünschtenfalls können Dispersionsmittel wie Propylenglykol oder ein Polyäthylenglykol zugesetzt werden.

Es war übrigens eine gelartige Salbe bereits bekannt (DE 2 854 221), welche als Desinfektionsmittel einen Alkohol enthält und zur äusserlichen Desinfektion insbesondere der Hände verwendet werden soll. Die gelartige Konsistenz wird durch eine Grundlage gewährleistet, welche aus Wasser, einem Carboxyvinylpolymeren und einer Base besteht.

Überraschenderweise wurde nun gefunden, dass bei Auslassen jeglichen Glykols oder Polyalkylenglykols in der oben beschriebenen Zusammensetzung eine gelartige Salbe erhalten wird, welche nicht nur Wirksamkeit bei der lokalen Anwendung aufweist, sondern in dieser Hinsicht das nächstverwandte Präparat sogar signifikant übertrifft.

Das neue Präparat enthält erfindungsgemäss Indomethacin, einen wasserlöslichen Celluloseäther oder/und eine Polyacrylsäure, deren Carboxygruppen durch ein wasserlösliches Amin neutralisiert sind, ferner Wasser und Äthanol oder Isopropanol. Die genannten Komponenten liegen unter Ausschluss eines Glykols oder eines Polyalkylenglykols, in für die Bildung eines Gels geeignetem Mengenverhältnis vor.

Der wasserlösliche Celluloseäther und die Polyacrylsäure fungieren im Präparat als Geliermittel, wobei die sauren Gruppen der letzteren durch die basische(n) Gruppe(n) des wasserlöslichen Amins neutralisiert werden sollen. Ein solches Amin kann z.B. Methylamin, Dimethylamin, Äthylamin, Diäthylamin, Propylamin, Isopropyl- amin, Diisopropylamin, Cyclohexylamin, Benzylamin, Guanidin, Pyrrolidin, Piperidin, Morpholin, Arginin, Lysin, Äthanolamin, Diäthanolamin, Diisopropanolamin oder Triäthanolamin sein. Bevorzugt werden die bei Raumtemperatur flüssigen niederen aliphatischen Amine, beispielsweise Diisopropanolamin oder Diäthylamin. Das Amin wird mit Vorteil im Überschuss gegenüber dem stöchiometrischen Verhältnis zu den Carboxygruppen der Polyacrylsäure verwendet.

Die Salbe soll einen physiologisch verträglichen pH-Wert aufweisen; sie wird mit Vorteil auf einen pH-Bereich von 6,7 bis 7,0, vorzugsweise auf pH 6,8, eingestellt. Zur Einstellung des pH-Wertes kann man die nötige Menge vom oben erwähnten wasserlöslichen Amin zugeben.

Unter den Polyacrylsäuren, auch Carboxyvinylpolymere genannt, kann man insbesondere solche verwenden, welche unter dem Namen Carbopol ® erhältlich sind, beispielsweise Carbopol ® 941, Carbopol ® 934 oder Carbopol ® 940 mit einem durchschnittlichen Molekulargewicht von 1 250 000, 3 000 000 und 4 000 000 resp. (Hersteller: Goodrich Chemical Co., Cleveland, OH/USA).

Als Beispiele der wasserlöslichen Celluloseäther seien erwähnt Methylcellulose, Äthylcellulose, Carboxymethylcellulose (auch Celluloseglykolat genannt), Hydroxyäthylcellulose, Hydroxypropylcellulose und Celluloseäthansulfonsäure. Siehe auch dazu Ullmanns Encyklopädie der technischen Chemie, 4. Auflage, Band 9, Seiten 192–212, Verlag Chemie, Weinheim BRD, 1975.

Mit Vorteil enthält die Salbe ausserdem ein rückfettendes Mittel, um die mögliche Austrocknung der Haut unter der Wirkung des Äthanols oder Isopropanols wettzumachen. Beispiele der zu verwendenden rückfettenden Mittel sind Äthylcaproat, Äthyllaurat, Diäthylsebacat und Diisopropyladipat; bevorzugt wird letzteres.

Vorzugsweise enthält das Präparat auf 1,0 Gewichtsteil Indomethacin
von 0,5 bis 2,0 Gewichtsteile Polyacrylsäure oder/und wasserlöslicher Celluloseäther
von 30 bis 50 Gewichtsteile Äthanol oder Isopropanol
von 1,0 bis 5,0 Gewichtsteile rückfettendes Mittel (von 1,0 bis 2,0 Gewichtsteile wasserlösliches Amin, wenn eine Polyacrylsäure verwendet wird) und so viel Wasser, dass 100 Gewichtsteile gebildet werden.

Gewünschtenfalls kann die Salbe ausserdem ein Geruchsverbesserungsmittel (flavouring agent) enthalten. Es eignen sich als solches insbesondere ätherische Öle, wie Lavendelöl, Thymianöl, die Citrusöle, z.B. Bergamottöl, Citronenöl, die Koniferennadelöle wie Cypressenöl, Fichtennadelöl und Latschenkieferöl, ferner Citronellöl, Eucalyptusöl, Campher, Thymol usw. Von den Koniferennadelölen kann man z.B. 0,04 Gew.%, von Campher oder Thymol z.B. 0,05 Gew.% zusetzen.

Sofern die erfindungsgemässe Zusammensetzung bewahrt bleibt, kann das Präparat zusätzlich ein weiteres oder weitere Mittel enthalten, welche bei lokaler Anwendung eine antiphlogistische oder entzündungshemmende Wirkung zu ergänzen oder zu unterstützen vermögen und in Form einer gelartigen Salbe durch die Haut absorbiert werden; als Beispiele davon seien Analgetica und Muskelrelaxantien genannt. Selbstverständlich fallen derartige neue Präparate ebenfalls in den Schutzbereich des vorliegenden Patentes.

Um die Wirksamkeit des Präparates zu prüfen, wurde die Bioverfügbarkeit (bioavailability) des Indomethacins nach äusserlicher Applikation der Salbe bestimmt, d.h. jene Menge Indomethacin, welche nach Absorption durch die Haut im Blut erscheint. Die Untersuchung wurde an 6 freiwilligen Probanden mit einer 1%igen Salbe gemäss vorliegendem Beispiel 3 durchgeführt; als Referenz- oder Vergleichspräparat wurde ein 1%iges Handelspräparat nach der DE-OS 2 827 018 in die Untersuchung einbezogen.

Die Probanden wurden vor Beginn des Versuches über Art, Zweck und Tragweite des Versuches aufgeklärt und haben alle ihr Einverständnis zur Teilnahme an der Prüfung abgegeben. Danach waren sie fachärztlich untersucht (mit ausführlichem Labor-, Drogen- und Alkoholscreening) und als gesund eingestuft worden.

Im Einzeldosis-Versuch wurde den Probanden im cross-over 3 g (was einem Indomethacin-Gehalt von 30 mg entspricht) des neuen Präparates bzw. des Vergleichspräparates auf eine genau definierte Stelle des Unterarms aufgetragen.

Nach einer Auslassphase von 7 Tagen erhielten die Probanden im cross-over das jeweils andere Präparat.

Zur Bestimmmung der Serumspiegel von Indomethacin wurde Blut entnommen unmittelbar vor Applikation der Präparate und 15 Min., 30 Min., 45 Min., 1 h, 1 h 30 Min., 2 h, 3 h, 4 h, 5 h, 8 h, 12 h, und 24 h danach.

Die Serumspiegel, ihre Mittelwerte, Standardabweichungen und mittleren Standardfehler sind in den Tabellen 1 und 2 angegeben, die pharmakokinetischen Parameter finden sich in den Tabellen 3 und 4.

Tabelle 1

Serumspiegel an Indomethacin aus dem neuen Präparat
Konzentration in ng/ml

| Zeit Proband | 0 h | 15 Min. | 30 Min. | 45 Min. | 1 h · | 1 h 30 Min. | 2 h | 3 h | 4 h | 5 h | 8 h | 12 h | 24 h |
|---|---|---|---|---|---|---|---|---|---|---|---|---|---|
| 1 | 0 | 0 | 7,3 | 6,9 | 8,3 | 3,5 | 7 | 2,7 | 5,1 | 5,3 | 3,2 | 4,9 | 2,5 |
| 2 | 1,4 | 0 | 8,3 | 4,02 | 0 | 0 | 7,2 | 3,8 | 16,2 | 7,3 | 5,7 | 0 | 1,7 |
| 3 | 1,2 | 2,7 | 0 | 0 | 0 | 0 | 0 | 0 | 0 | 0 | 0 | 0 | 10,1 |
| 4 | (1,00 | 5,4 | 0 | – | 7,2 | 0 | 8,3 | 11,1 | 13,4 | 11,5 | 13 | 6 | 6,4 |
| 5 | 5,2 | 5,3 | 1,6 | 4,9 | 2,8 | 1,9 | 4,1 | 1,4 | 2,5 | 3,4 | 3,4 | 5,6 | 4,9 |
| 6 | 2,6 | 4,5 | 4,7 | 1,5 | 1,3 | 2 | 3,7 | (1,00 | 2,2 | 4,4 | 4 | 4,6 | 1,5 |

Tabelle 1 (Fortsetzung)

Serumspiegel an Indomethacin aus dem neuen Präparat
Konzentration in ng/ml

| Zeit Pro-band | 0 h | 15 Min. | 30 Min. | 45 Min. | 1 h | 1 h 30 Min. | 2 h | 3 h | 4 h | 5 h | 8 h | 12 h | 24 h |
|---|---|---|---|---|---|---|---|---|---|---|---|---|---|
| mean | 2,08 | 2,98 | 3,65 | 3,46 | 3,27 | 1,23 | 5,05 | 3,8 | 6,57 | 5,32 | 4,88 | 3,52 | 4,52 |
| sdev | 1,97 | 2,51 | 3,66 | 2,74 | 3,64 | 1,47 | 3,07 | 4,32 | 6,64 | 3,87 | 4,39 | 2,77 | 3,35 |
| sem | 0,88 | 1,02 | 1,49 | 1,23 | 1,49 | 0,6 | 1,25 | 1,93 | 2,71 | 1,58 | 1,79 | 1,13 | 1,37 |

mean: Mittelwert
sdev: Standardabweichung
sem: Standardfehler des Mittelwertes
( = Wert unterhalb der Nachweisgrenze
– = Probe verloren oder zerstört

Tabelle 2

Serumspiegel an Indomethacin aus dem Referenzpräparat
Konzentration in ng/ml

| Zeit Pro-band | 0 h | 15 Min. | 30 Min. | 45 Min. | 1 h | 1 h 30 Min. | 2 h | 3 h | 4 h | 5 h | 8 h | 12 h | 24 h |
|---|---|---|---|---|---|---|---|---|---|---|---|---|---|
| 1 | 0 | 2 | 2,6 | 6,2 | 1,4 | – | 1,3 | 2,7 | 0 | 0 | 0 | 6,8 | – |
| 2 | – | 0 | (1,00 | 5,9 | 2,7 | 2,3 | 2,2 | (1,00 | 1,5 | 0 | 0 | 0 | 6,3 |
| 3 | 5,2 | 8,5 | 2,8 | 3,3 | 2,3 | 5,2 | 2,2 | (1,00 | 3,1 | 0 | 0 | – | 0 |
| 4 | – | 2,6 | (1,00 | 0 | 2,5 | 0 | (1,00 | 0 | 1,9 | (1,00 | 2,8 | 6 | 0 |
| 5 | 0 | 7,8 | 2,9 | 6,7 | 7,1 | 5 | 5 | 6,1 | 0 | 7,3 | 7,2 | 8,9 | 0 |
| 6 | 0 | 1,7 | 6 | 1,1 | 0 | 1 | 1 | (1,00 | 1,9 | 1 | 2,5 | 4,3 | 0 |
| mean | 1,3 | 3,77 | 3,58 | 3,87 | 2,67 | 2,7 | 2,34 | 2,93 | 1,4 | 1,66 | 2,08 | 5,2 | 1,26 |
| sdev | 2,6 | 3,51 | 1,62 | 2,85 | 2,39 | 2,34 | 1,58 | 3,06 | 1,21 | 3,18 | 2,82 | 3,34 | 2,82 |
| sem | 1,3 | 1,43 | 0,81 | 1,16 | 0,98 | 1,05 | 0,71 | 1,76 | 0,49 | 1,42 | 1,15 | 1,5 | 1,26 |

mean: Mittelwert
sdev: Standardabweichung
sem: Standardfehler des Mittelwertes
( = Wert unterhalb der Nachweisgrenze
– = Probe verloren oder zerstört

Tabelle 3
Pharmakokinetische Parameter des neuen
Präparates, berechnet aus den Serumspiegeln
(Tabelle 1)

| Proband | AUC | Cmax | Tmax |
|---|---|---|---|
| 1 | 97,46 | 8,3 | 1 |
| 2 | 73,41 | 16,2 | 4 |
| 3 | 61,43 | 10,1 | 24 |
| 4 | 189,9 | 13,4 | 4 |
| 5 | 105,5 | 5,6 | 12 |
| 6 | 81,01 | 4,7 | 0,5 |
| mean | 101,4 | 9,717 | 7,583 |
| stdev | 46,18 | 4,469 | 9,036 |
| sem | 18,85 | 1,825 | 3,689 |
| Einheit: | hxng/ml | ng/ml | h |

AUC: Fläche unter der Kurve
Cmax: Maximale Konzentration
Tmax: Zeitpunkt der maximalen Konzentration

Tabelle 4
Pharmakokinetische Parameter des
Referenzpräparates, berechnet nach den
Serumspiegeln (Tabelle 2)

| Proband | AUC | Cmax | Tmax |
|---|---|---|---|
| 1 | 21,18 | 6,8 | 12 |
| 2 | 47,18 | 6,3 | 24 |
| 3 | 15,16 | 8,5 | 0,25 |
| 4 | 65,54 | 6 | 12 |
| 5 | 130,4 | 8,9 | 12 |
| 6 | 51,95 | 6 | 0,5 |
| mean | 55,23 | 7,083 | 10,13 |
| stdev | 41,45 | 1,292 | 8,868 |
| sem | 16,92 | 0,5275 | 3,62 |
| Einheit: | hxng/ml | ng/ml | h |

AUC: Fläche unter der Kurve
Cmax: Maximale Konzentration
Tmax: Zeitpunkt der maximalen Konzentration

Wie aus den Tabellen 3 und 4 ersichtlich, ergab sich für das neue Präparat ein Flächenwert von 101,4 h × ng/ml, für das Referenzpräparat ergab sich ein Wert von 55,2 h × ng/ml. Die maximale Konzentration für das neue Präparat lag bei 9,7 ng/ml, für das Referenzpräparat bei 7,1 ng/ml.

Die relative Bioverfügbarkeit des erfindungsgemässen Präparates gegenüber dem Referenzpräparat beträgt 183,6%, sie ist also um einen Faktor 1,8 besser.

Ein Vergleich der Zusammensetzung der beiden Präparate zeigt, dass dieser Unterschied in der Wirksamkeit nur durch die Auslassung des Glykols oder Polyalkylenglykols bedingt sein kann. Dieses Ergebnis war umso unerwarteter, als z.B. Propylenglykol häufig in äusserlich applizierten Arzneimitteln als Lösungsmittel für Wirkstoffe eingesetzt wird und dabei den gelösten Wirkstoff durch die Hornschichtbarriere der Haut mitschleppt (M. Gloor, Pharmakologie dermatologischer Externa, Springer-Verlag, Berlin Heidelberg New York 1982, Seiten 7–8 und 42). Übrigens werden Propylenglykol und Polyäthylenglykol üblicherweise als wasserlösliche Grundlagen für Salben verwendet (L.S. Goodman und A. Gilman, The Pharmacological Basis of Therapeutics, 5. Auflage, Macmillan Publishing Co. Inc., New York 1975, Seite 947), und dies nicht nur für Salben anderer Wirkstoffzusammensetzung, sondern eben auch für Indomethacin enthaltende Salben (Europäische Patentanmeldung 55 029).

Die therapeutische Wirkung des neuen Präparates kann, eher als durch pharmakologische Versuche in vitro oder am lebenden Tier, am besten an menschlichen Patienten geprüft werden. Erst der therapeutischen Anwendung in den konkreten Fällen des täglichen Lebens und der Beurteilung der Ergebnisse durch die behandelnden Ärzte kommt in bezug auf die Wirksamkeit endgültiger Aussagewert zu.

Deshalb wurden die Wirksamkeit und die Verträglichkeit des Präparates gemäss Beispiel 4 (unter dem geschützten Markennamen Bonidon Gel ®) in einer seit Januar 1983 laufenden multizentrischen Phase-IV-Praxisstudie bei der Behandlung von entzündlichen und degenerativen rheumatischen Erkrankungen und Traumata der Gelenke und Weichteile an 153 Patienten untersucht.

Dabei sollte Bonidon Gel ® entsprechend der Gebrauchsinformation 2–3 mal täglich grossflächig dünn auf die betroffenen Körperteile aufgetragen werden. Die Therapie sollte nicht länger als 3 Wochen dauern. Der Krankheitsverlauf war nach der 1, 2 und gegebenenfalls nach 3 Wochen zu kontrollieren. Der Grad der Beschwerden wurde vom Arzt beurteilt und nach den Stufen schwer, mittel, leicht und beschwerdefrei im Protokollbogen eingetragen.

Wegen Nichteinhalten der Prüfungsbedingungen mussten 24 Patienten ausgeschlossen werden, bei einem weiteren Patienten lag eine ungenügende Datenerfassung vor, einer brach wegen unerwünschter Arzneimittelwirkung die Behandlung ab; für den Bericht wurden also die Daten von 127 Patienten ausgewertet. Von diesen litten 39 unter degenerativen Gelenkerkrankungen, 16 unter Weichteilrheumatismus, 43 Patienten erlitten traumatische Verletzungen und 29 litten unter sonstigen Beschwerden.

Beurteilt wurden die Parameter Schmerz, Schwellung und Funktionseinschränkung. 19% der Patienten wurden eine Woche lang, 33,2% zwei Wochen lang und 47,8% drei Wochen lang behandelt. Bei Therapieende waren in 62,2% der Fälle die Beschwerden völlig verschwunden, in 33,4% hatten sich die Symptome gebessert.

Die Verträglichkeit war sehr gut. Lediglich 4,7% der Patienten berichteten über Juckreiz, Rötung oder Brennen der behandelten Hautflächen, wobei die unerwünschten Wirkungen überwiegend als leicht und flüchtig bezeichnet wurden.

Im folgenden werden Beispiele der Zusammensetzung und der Herstellung des erfindungsgemässen Präparates gegeben; die Mengenangaben beziehen sich auf Gewichtsteile.

Tabelle 5

| Nr. | Substanz | 1 | 2 | 3 | 4 |
|---|---|---|---|---|---|
| 1 | Indomethacin | 1,0 | 1,0 | 1,0 | 1,0 |
| 2 | Carbopol (R) 941 | – | 1,0 | 1,5 | 2,0 |
| 3 | Hydroxypropyl-cellulose Klucel (R)* | 2,0 | 0,5 | – | – |
| 4 | Diisopropanolamin | 1,0 | 1,5 | 1,8 | 2,0 |
| 5 | Äthanol 95% | 40,0 | 40,0 | 35,0 | 50,0 |
| 6 | Diisopropyladipat | 3,0 | 4,0 | 5,0 | 5,0 |
| 7 | Gereinigtes Wasser ad | 100,0 | 100,0 | 100,0 | 100,0 |

* Hersteller: Hercules Inc., Wilmington (DE/USA)

Beispiel 1

– Man löst (4) in 10 Teilen Wasser (7)    Lösung A

– Man mischt (5) und (6) miteinander, löst (1) darin unter Rühren und gibt langsam so viel der Lösung A hinzu, dass ein pH von 6,9 erreicht wird    Lösung B

– Man mischt (3) mit 20 Teilen Wasser (7), stellt daraus eine homogene Suspension her, gibt langsam die Lösung B hinzu und rührt gründlich    Gel C

– Man lässt das Gel über Nacht stehen und bringt schliesslich auf das gewünschte Gewicht durch Zugabe von Wasser (7)    Gel D

Beispiel 2

– Man löst (4) in 10 Teilen Wasser (7)    Lösung A

– Man mischt (5) und (6) miteinander, löst (1) darin unter Rühren und gibt langsam so viel der Lösung

A hinzu, dass ein pH von 6,8 erreicht wird     **Lösung B**

- Man mischt (3) mit 20 Teilen Wasser (7), stellt daraus eine homogene Suspension her, gibt langsam die Lösung B hinzu, mischt gründlich und lässt über Nacht stehen     **Gel C**

- Man löst (2) in 20 Teilen Wasser (7), lässt während einiger Zeit aufquellen, rührt bis zur Bildung einer homogenen Masse, mischt diese langsam und unter ständigem Rühren in das Gel C hinein, um ein einheitliches Gel zu bilden, stellt das pH des Gels durch langsame Zugabe der Lösung A auf 6,8 und bringt schliesslich auf das gewünschte Gewicht durch Zugabe von Wasser (7)     **Gel D**

**Beispiel 3 und 4**

- Man löst (4) in 10 Teilen Wasser (7)     **Lösung A**
- Man löst (1) in einem Gemisch von (5) und (6) unter Bildung einer klaren Lösung und gibt langsam so viel der Lösung A hinzu, dass ein pH von 6,8 erreicht wird     **Lösung B**
- Man mischt (2) mit 20 Teilen Wasser (7), lässt während einiger Zeit aufquellen, rührt bis zur Bildung einer homogenen Masse und gibt unter Rühren Lösung A hinzu, bis das pH auf 6,8 eingestellt ist     **Gel C**
- Man mischt die Lösung B langsam und unter ständigem Rühren in das Gel C hinein, um ein einheitliches Gel zu bilden, und bringt schliesslich auf das gewünschte Gewicht durch Zugabe von Wasser (7)     **Gel D**

**Beispiel 5**

- Man löst 2,0 g Diisopropanolamin in 10 ml gereinigtes Wasser     **Lösung A**
- Man löst 1,0 g Indomethacin in einem Gemisch von 50 g Isopropanol und 5 g Diisopropyladipat unter Bildung einer klaren Lösung und gibt langsam so viel der Lösung A hinzu, dass ein pH von 6,9 erreicht wird     **Lösung B**
- Man mischt 2,0 g Carboxymethylcellulose mit 20 ml gereinigtem Wasser, lässt während einiger Zeit aufquellen, rührt bis zur Bildung einer homogenen Masse und gibt unter Rühren die Lösung A hinzu, bis das pH auf 6,9 eingestellt ist     **Gel C**
- Man mischt die Lösung B langsam und unter ständigem Rühren in das Gel C hinein, um ein einheitliches Gel zu bilden, und bringt schliesslich auf ein Gewicht von 100 g durch Zugabe von gereinigtem Wasser     **GEL D**

**Beispiel 6**

- Man löst 2,0 g Diisopropanolamin in 10 ml gereinigtes Wasser     **Lösung A**
- Man löst 1,0 g Indomethacin in einem Gemisch von 50 g Isopropanol, 5 g Diisopropyladipat und 0,07 g Fichtennadelöl unter Bildung einer klaren Lösung und gibt langsam so viel der Lösung A hinzu, dass ein pH von 6,9 erreicht wird     **Lösung B**

Die weiteren Schritte werden genau wie im Beispiel 5 durchgeführt und führen zu 100 g Gel, das bis auf den Geruch und den Gehalt an Fichtennadelöl (0,04 Gew.%) jenem gemäss Beispiel 5 entspricht.

**Patentansprüche für die Vertragsstaaten BE DE FR GB IT LU NL SE**

1. Indomethacin enthaltendes Präparat in Form einer gelartigen Salbe zur lokalen Behandlung von Entzündungen, dadurch gekennzeichnet, dass es, nebst Indomethacin selbst, als Geliermittel einen wasserlöslichen Celluloseäther oder/und eine Polyacrylsäure, deren Carboxygruppen durch ein wasserlösliches Amin neutralisiert sind, Wasser und Äthanol oder Isopropanol enthält und die genannten Komponenten in zur Bildung eines Gels geeignetem Mengenverhältnis und unter Ausschluss eines Glykols oder eines Polyalkylenglykols vorliegen.

2. Präparat nach Anspruch 1, dadurch gekennzeichnet, dass es ausserdem ein rückfettendes Mittel enthält.

3. Präparat nach Anspruch 1 oder 2, dadurch gekennzeichnet, dass als wasserlöslicher Celluloseäther Methylcellulose, Äthylcellulose, Carboxymethylcellulose, Hydroxyäthylcellulose, Hydroxypropylcellulose oder Celluloseäthansulfonsäure verwendet wird.

4. Präparat nach Anspruch 1 oder 2, dadurch gekennzeichnet, dass als wasserlösliches Amin zur Neutralisierung der Polyacrylsäure ein bei Raumtemperatur flüssiges, niederes aliphatisches Amin verwendet wird.

5. Präparat nach Anspruch 2, dadurch gekennzeichnet, dass als rückfettendes Mittel eine Verbindung aus der Gruppe Diisopropyladipat, Diäthylsebacat, Äthylcaproat und Äthyllaurat verwendet wird.

6. Präparat nach Anspruch 5, dadurch gekennzeichnet, dass als rückfettendes Mittel Diisopropyladipat verwendet wird.

7. Präparat nach Anspruch 1, dadurch gekennzeichnet, dass es auf 1,0 Gewichtsteil Indomethacin 0,5 bis 2,0 Gewichtsteile wasserlöslichen Celluloseäther oder/und Polyacrylsäure, das wasserlösliche Amin in höherem als das stöchiometrische Verhältnis zu den Carboxygruppen der Polyacrylsäure, 30 bis 50 Gewichtsteile Äthanol oder Isopropanol, 1,0 bis 5,0 Gewichtsteile rückfettendes Mittel und das Komplement an Wasser bis auf 100 Gewichtsteile enthält.

8. Präparat nach Anspruch 1, dadurch gekennzeichnet, dass es ausserdem ein Analgeticum enthält, welches bei lokaler Anwendung in Form einer gelartigen Salbe durch die Haut absorbiert wird.

9. Präparat nach Anspruch 1, dadurch gekennzeichnet, dass es ausserdem ein Muskelrelaxans enthält, welches bei lokaler Anwendung in Form einer gelartigen Salbe durch die Haut absorbiert wird.

**Patentansprüche für den Vertragsstaat AT**

1. Verfahren zur Herstellung eines Indomethacin enthaltenden Präparates in Form einer gelartigen Salbe zur lokalen Behandlung von Entzündungen, dadurch gekennzeichnet, dass man Indomethacin selbst, als Geliermittel einen wasserlöslichen Celluloseäther oder/und eine Polyacrylsäure, deren Carboxgruppen durch ein wasserlösliches Amin neutralisiert sind, Wasser und Äthanol oder Isopropanol in zur Bildung eines Gels geeignetem Mengenverhältnis und unter Auslassung eines Glykols oder eines Polyalkylenglykols miteinander vermischt.

2. Verfahren nach Anspruch 1, dadurch gekennzeichnet, dass man ausserdem ein rückfettendes Mittel hinzumischt.

3. Verfahren nach Anspruch 1 oder 2, dadurch gekennzeichnet, dass als wasserlöslicher Celluloseäther Methylcellulose, Äthylcellulose, Carboxymethylcellulose, Hydroxyäthylcellulose, Hydroxypropylcellulose oder Celluloseäthansulfonsäure verwendet wird.

4. Verfahren nach Anspruch 1 oder 2, dadurch gekennzeichnet, dass als wasserlösliches Amin zur Neutralisierung der Polyacrylsäure ein bei Raumtemperatur flüssiges, niederes aliphatisches Amin verwendet wird.

5. Verfahren nach Anspruch 2, dadurch gekennzeichnet, dass als rückfettendes Mittel eine Verbindung aus der Gruppe Diisopropyladipat, Diäthylsebacat, Äthylcaproat und Äthyllaurat verwendet wird.

6. Verfahren nach Anspruch 5, dadurch gekennzeichnet, dass als rückfettendes Mittel Diisopropyladipat verwendet wird.

7. Verfahren nach Anspruch 1, dadurch gekennzeichnet, dass man auf 1,0 Gewichtsteil Indomethacin 0,5 bis 2,0 Gewichtsteile wasserlöslichen Celluloseäther oder/und Polyacrylsäure, das wasserlösliche Amin in höherem als das stöchiometrische Verhältnis zu den Carboxgruppen der Polyacrylsäure, 30 bis 50 Gewichtsteile Äthanol oder Isopropanol, 1,0 bis 5,0 Gewichtsteile rückfettendes Mittel und das Komplement an Wasser bis auf 100 Gewichtsteile miteinander vermischt.

8. Verfahren nach Anspruch 2, dadurch gekennzeichnet, dass man Diisopropanolamin in Wasser unter Bildung einer Lösung A auflöst, 95%iger Äthanol und Diisopropyladipat miteinander mischt, Indomethacin darin auflöst und so viel der Lösung A hinzugibt, dass unter Bildung einer Lösung B ein pH-Wert von 6,9 erreicht wird, Hydroxypropylcellulose mit Wasser unter Bildung einer homogenen Suspension mischt und die Lösung B unter Bildung eines Gels hinzugibt, das Gel über Nacht stehen lässt und durch Zugabe von Wasser auf das gewünschte Endgewicht bringt.

9. Verfahren nach Anspruch 2, dadurch gekennzeichnet, dass man Diisopropanolamin in Wasser unter Bildung einer Lösung A auflöst, 95%iger Äthanol und Diisopropyladipat miteinander mischt, Indomethacin darin auflöst und so viel der Lösung A hinzugibt, dass unter Bildung einer Lösung B ein pH-Wert von 6,8 erreicht wird, Hydroxypropylcellulose mit Wasser unter Bildung einer homogenen Suspension mischt, die Lösung B hinzugibt und unter Bildung eines Gels C über Nacht stehen lässt, Polyacrylsäure mit Wasser unter Bildung einer homogenen Masse mischt, diese Masse in das Gel C hineinmischt und so viel der Lösung A hinzugibt, dass ein pH-Wert von 6,8 erreicht wird, und durch Zugabe von Wasser auf das gewünschte Endgewicht bringt.

10. Verfahren nach Anspruch 2, dadurch gekennzeichnet, dass man Diisopropanolamin in Wasser unter Bildung einer Lösung A auflöst, 95%iger Äthanol und Diisopropyladipat miteinander mischt, Indomethacin darin auflöst und so viel der Lösung A hinzugibt, dass unter Bildung einer Lösung B ein pH-Wert von 6,8 erreicht wird, Polyacrylsäure mit Wasser unter Bildung einer homogenen Masse mischt und soviel der Lösung A hinzugibt, dass unter Bildung eines Gels C ein pH-Wert von 6,8 erreicht wird, die Lösung B in das Gel C hineinmischt und durch Zugabe von Wasser auf das gewünschte Endgewicht bringt.

11. Verfahren nach Anspruch 2, dadurch gekennzeichnet, dass man Diisopropanolamin in Wasser unter Bildung einer Lösung A auflöst, Isopropanol und Diisopropyladipat gegebenenfalls unter Zugabe von Fichtennadelöl miteinander mischt, Indomethacin darin auflöst und soviel der Lösung A hinzugibt, dass unter Bildung einer Lösung B ein pH-Wert von 6,9 erreicht wird, Carboxymethylcellulose mit Wasser unter Bildung einer homogenen Masse mischt und soviel der Lösung A hinzugibt, dass unter Bildung eines Gels C ein pH-Wert von 6,9 erreicht wird, die Lösung B in das Gel C hineinmischt und durch Zugabe von Wasser auf das gewünschte Endgewicht bringt.

**Revendications pour les Etats contractants BE DE FR GB IT LU NL SE**

1. Préparation contenant de l'indométhacine, sous forme de gel, pour le traitement local d'inflammations, caractérisée en ce que, en plus de l'indométhacine elle-même, elle contient comme agent gélifiant un éther de cellulose soluble dans l'eau ou/et un acide polyacrylique dont les groupes carboxy sont neutralisés par une amine soluble dans l'eau, de l'eau et de l'éthanol ou de l'isopropanol et que lesdits composants sont présents en une proportion appropriée à la formation d'un gel et à l'exclusion d'un glycol ou d'un polyalkylène glycol.

2. Préparation selon la revendication 1, caractérisée en ce qu'elle contient en outre un agent lubrifiant.

3. Préparation selon la revendication 1 ou 2, caractérisée en ce que l'on utilise comme éther de cellulose soluble dans l'eau la méthylcellulose, l'éthylcellulose, la carboxyméthylcellulose, l'hydroxyéthylcellulose, l'hydroxypropylcellulose ou l'acide celluloseéthane sulfonique.

4. Préparation selon la revendication 1 ou 2, caractérisée en ce que l'on utilise comme amine soluble dans l'eau, pour la neutralisation de l'acide polyacrylique, une amine aliphatique inférieure, liquide à la température ordinaire.

5. Préparation selon la revendication 2, caractérisée en ce que l'on utilise comme agent lubrifiant un composé choisi dans le groupe consistant en l'adipate de diisopropyle, le sébacate de diéthyle, le caproate d'éthyle et le laurate d'éthyle.

6. Préparation selon la revendication 5, caractérisée en ce que l'on utilise comme agent lubrifiant l'adipate de diisopropyle.

7. Préparation selon la revendication 1, caractérisée en ce qu'elle contient, pour 1,0 partie en poids d'indométhacine, de 0,5 à 2,0 parties en poids d'un éther de cellulose soluble dans l'eau ou/et d'acide polyacrylique, l'amine soluble dans l'eau en une proportion, par rapport aux groupes carboxy de l'acide polyacrylique, supérieure à la proportion stoechiométrique, de 30 à 50 parties en poids d'éthanol ou d'isopropanol, de 1,0 à 5,0 parties en poids de l'agent lubrifiant, et de l'eau pour compléter à 100 parties en poids.

8. Préparation selon la revendication 1, caractérisée en ce qu'elle contient en outre un analgésique qui, en application locale sous forme d'un gel, est absorbé par la peau.

9. Préparation selon la revendication 1, caractérisée en ce qu'elle contient en outre un myorelaxant qui, en application locale sous forme d'un gel, est absorbé par la peau.

**Revendications pour l'Etat contractant AT**

1. Procédé de fabrication d'une préparation contenant de l'indométhacine, sous forme de gel, pour le traitement local d'inflammations, caractérisé en ce que l'on mélange l'indométhacine elle-même, comme agent gélifiant un éther de cellulose soluble dans l'eau ou/et un acide polyacrylique dont les groupes carboxy sont neutralisés par une amine soluble dans l'eau, de l'eau et de l'éthanol ou de l'isopropanol en une proportion appropriée à la formation d'un et à l'exclusion d'un glycol ou d'un polyalkylène glycol.

2. Procédé selon la revendication 1, caractérisée en ce que l'on ajoute et mélange en outre un agent lubrifiant.

3. Procédé selon la revendication 1 ou 2, caractérisé en ce que l'on utilise comme éther de cellulose soluble dans l'eau la méthylcellulose, l'éthylcellulose, la carboxyméthylcellulose, l'hydroxyéthylcellulose, l'hydroxypropylcellulose ou l'acide celluloseéthane sulfonique.

4. Procédé selon la revendication 1 ou 2, caractérisé en ce que l'on utilise comme amine soluble dans l'eau, pour la neutralisation de l'acide polyacrylique, une amine aliphatique inférieure, liquide à la température ordinaire.

5. Procédé selon la revendication 2, caractérisé en ce que l'on utilise comme agent lubrifiant un composé choisi dans le groupe consistant en l'adipate de diisopropyle, le sébacate de diéthyle, le caproate d'éthyle et le laurate d'éthyle.

6. Procédé selon la revendication 5, caractérisé en ce que l'on utilise comme agent lubrifiant l'adipate de diisopropyle.

7. Procédé selon la revendication 1, caractérisé en ce que l'on mélange, pour 1,0 partie en poids d'indométhacine, de 0,5 à 2,0 parties en poids d'un éther de cellulose soluble dans l'eau ou/et d'acide polyacrylique, l'amine soluble dans l'eau en une proportion, par rapport aux groupes carboxy de l'acide polyacrylique, supérieure à la proportion stoechiométrique, de 30 à 50 parties en poids d'éthanol ou d'isopropanol, de 1,0 à 5,0 parties en poids d'agent lubrifiant et le complément en eau pour 100 parties en poids.

8. Procédé selon la revendication 2, caractérisé en ce que l'on dissout de la diisopropanolamine dans de l'eau, ce par quoi il se forme une solution A, on mélange entre eux de l'éthanol à 95% et de l'adipate de diisopropyle, on dissout l'indométhacine dans ce mélange et l'on y ajoute suffisamment de la solution A pour parvenir, dans la solution B ainsi formée, à une valeur de pH de 6,9, on mélange de l'hydroxypropylcellulose avec de l'eau en formant ainsi une suspension homogène et l'on y ajoute la solution B, ce qui forme un gel, on abandonne le gel pendant la nuit et on l'amène, par addition d'eau, au poids final désiré.

9. Procédé selon la revendication 2, caractérisé en ce que l'on dissout de la diisopropanolamine dans de l'eau, ce par quoi il se forme une solution A, on mélange l'un avec l'autre de l'éthanol à 95% et de l'adipate de diisopropyle, on dissout l'indométhacine dans ce mélange et l'on y ajoute suffisamment de solution A pour obtenir, dans la solution B ainsi formée, une valeur de pH de 6,8, on mélange de l'hydroxypropylcellulose avec de l'eau, formant ainsi une suspension homogène, on y ajoute la solution B et on abandonne pendant la nuit, ce par quoi il se forme un gel C, on mélange de l'acide polyacrylique avec de l'eau en formant une masse homogène, on fait entrer cette masse dans le gel C en mélangeant et on y ajoute suffisamment de solution A pour obtenir une valeur de pH de 6,8 et l'on amène, par addition d'eau, au poids final désiré.

10. Procédé selon la revendication 2, caractérisé en ce que l'on dissout de la diisopropanolamine dans de l'eau, ce par quoi il se forme une solution A, on mélange l'un avec l'autre de l'éthanol à 95% et de l'adipate de diisopropyle, on dissout l'indométhacine dans ce mélange et l'on y ajoute suffisamment de solution A pour obtenir, dans la solution B ainsi formée, une valeur de pH de 6,8, on mélange de l'acide polyacrylique avec de l'eau, en formant ainsi une masse homogène, et on ajoute suffisamment de solution A pour obtenir une valeur de pH de 6,8 dans le gel C ainsi formé, on fait entrer la solution B dans le gel C en mélangeant et l'on amène, par addition d'eau, au poids final désiré.

11. Procédé selon la revendication 2, caractérisé en ce que l'on dissout de la diisopropanolamine dans de l'eau, ce par quoi il se forme une solution A, on mélange l'un avec l'autre de l'isopropanol et de l'adipate de diisopropyle, cas échéant avec addition d'essence de pin sylvestre, on dissout l'indométhacine dans ce mélange et l'on y ajoute suffisamment de solution A pour obtenir, dans la solution B ainsi formée, une valeur de pH de 6,9, on mélange de la carboxyméthylcellulose avec de l'eau, en formant ainsi une masse homogène, et l'on y ajoute suffisamment de solution A pour obtenir, dans le gel C ainsi formé, une valeur de pH de 6,9, on fait entrer la solution B dans le gel C en mélangeant et l'on amène, par addition d'eau, au poids final désiré.

**Claims for the contracting states BE DE FR GB IT LU NL SE**

1. Indomethacin-containing preparation in the form of a gel-like ointment for the local treatment of inflammation, characterised in that it contains, in addition to indomethacin itself, as the gelatinising agent a water-soluble cellulose ether and/or a polyacrylic acid the carboxy groups of which are neutralised by a water-soluble amine, and water and ethanol or isopropanol and the above-mentioned components are present in a proportion suitable for the formation of a gel and with the exclusion of any glycol or polyalkyleneglycol.

2. Preparation as claimed in claim 1, characterised in that it additionally contains a re-fatting agent.

3. Preparation as claimed in claim 1 or 2, characterised in that methylcellulose, ethylcellulose, carboxymethylcellulose, hydroxyethylcellulose, hydroxypropylcellulose or cellulose ethanesulphonic acid is used as the water soluble cellulose ether.

4. Preparation as claimed in claim 1 or 2, characterised in that a lower aliphatic amine which is liquid at ambient temperature is used as the water-soluble amine for neutralising the polyacrylic acid.

5. Preparation as claimed in claim 2, characterised in that a compound selected from the group comprising diisopropyladipate, diethylsebacate, ethylcaproate and ethyllaurate is used as the re-fatting agent.

6. Preparation as claimed in claim 5, characterised in that diisopropyladipate is used as the re-fatting agent.

7. Preparation as claimed in claim 1, characterised in that it contains, to 1.0 part by weight of indomethacin, 0.5 to 2.0 parts by weight of water-soluble cellulose ether and/or polyacrylic acid, the watersoluble amine in a higher than stoichiometric ratio to the carboxy groups of the polyacrylic acid, 30 to 50 parts by weight of ethanol or isopropanol, 1.0 to 5.0 parts by weight of re-fatting agent and sufficient water to make it up to 100 parts by weight.

8. Preparation as claimed in claim 1, characterised in that it also contains an analgesic which is absorbed through the skin when applied topically in the form of a gel-like ointment.

9. Preparation as claimed in claim 1, characterised in that it also contains a muscle relaxant which is absorbed through the skin when applied topically in the form of a gel-like ointment.

**Claims for the contracting state: AT**

1. Process for preparing an indomethacin-containing preparation in the form of a gel-like ointment for the local treatment of inflammation, characterised in that indomethacin itself, and as a gelatinising agent a water-soluble cellulose ether and/or a polyacrylic acid, the carboxy groups of which are neutralised by a water-soluble amine, and water and ethanol or isopropanol are mixed together in a proportio in suitable for the formation of a gel and with the omission of a glycol or a polyalkyleneglycol.

2. Process as claimed in claim 1, characterised in that a re-fatting agent is a also added.

3. Process as claimed in claim 1 or 2, characterised in that methylcellulose, ethylcellulose, carboxymethylcellulose, hydroxyethylcellulose, hydroxypropylcellulose or cellulose ethanesulphonic acid is used as the water soluble cellulose ether.

4. Process as claimed in claim 1 or 2, characterised in that a lower aliphatic amine which is liquid at ambient temperature is used as the water soluble amine for neutralising the polyacrylic acid.

5. Process as claimed in claim 2, characterised in that a compound selected from the group comprising diisopropyladipate, diethylsebacate, ethylcaproate and ethyllaurate is used as the re-fatting agent.

6. Process as claimed in claim 5, characterised in that diisopropyladipate is used as the re-fatting agent.

7. Process as claimed in claim 1, characterised in that, to 1.0 part by weight of indomethacin, 0.5 to 2.0 parts by weight of water soluble cellulose ether and/or polyacrylic acid, the water-soluble amine in a higher than stoichiometric ratio to the carboxy groups of the polyacrylic acid, 30 to 50 parts by weight of ethanol or isopropanol, 1.0 to 5.0 parts by weight of re-fatting agent and sufficient water to make it up to 100 parts by weight are mixed together.

8. Process as claimed in claim 2, characterised in that diisopropanolamine is dissolved in water to form a solution A, 95% ethanol and diisopropyladipate are mixed together, indomethacin is dissolved therein and a sufficient quantity of solution A is added to give a pH value of 6,9, while forming a solution B, hydroxypropylcellulose is mixed with water to form a homogeneous suspension and solution B is added to form a gel, the gel is left to stand overnight and brought to the desired finished weight by the addition of water.

9. Process as claimed in claim 2, characterised in that diisopropanolamine is dissolved in water to form a solution A, 95% ethanol and diisopropyladipate are mixed together, indomethacin is dissolved therein and a sufficient quantity of solution A is added to give a pH value of 6,8, forming a solution B, hydroxypropylcellulose is mixed with water to form a homogeneous suspension, the solution B is added and left to stand overnight, forming a gel C, polyacrylic acid is mixed with water to form a homogeneous mass, this mass is mixed into the gel C and a sufficient amount of solution A is added to give a pH value of 6,8, and the desired finished weight is achieved by the addition of water.

10. Process as claimed in claim 2, characterised in that diisopropanolamine is dissolved in water to form a solution A, 95% ethanol and diisopropyladipate are mixed together, indomethacin is dissolved therein and a sufficient quantity of solution A is added to give a pH value of 6.8, forming a solution B, polyacrylic acid is mixed with water to form a homogeneous mass and a sufficient amount of solution A is added to give a pH of 6.8, forming a gel C, solution B is mixed into gel C and this is adjusted to the desired finished weight by the addition of water.

11. Process as claimed in claim 2, characterised in that diisopropanolamine is dissolved in water to form a solution A, isopropanol and diisopropyladipate are mixed together, optionally with the addition of pine needle oil, indomethacin is dissolved therein and a sufficient amount of solution A is added to give a pH of 6.9, forming a solution B, carboxymethylcellulose is mixed with water to form a homogeneous mass and a sufficient amount of solution A is added to give a pH of 6.9, forming a gel C, solution B is mixed into gel C and this is adjusted to the desired finished weight by the addition of water.